(19) Europäisches Patentamt
European Patent Office
Office européen des brevets

(11) EP 2 108 366 B1

(12) # EUROPEAN PATENT SPECIFICATION

(45) Date of publication and mention of the grant of the patent:
**19.10.2011 Bulletin 2011/42**

(51) Int Cl.:
***A61K 31/122*** *(2006.01)* ***A61P 11/00*** *(2006.01)*
***A61P 21/00*** *(2006.01)*

(21) Application number: **08007069.1**

(22) Date of filing: **09.04.2008**

(54) **Idebenone for the treatment of respiratory illness in muscular dystrophy**

Idebenon zur Behandlung von durch muskuläre Dystrophie vermittelten Atemwegserkrankungen

Idébenone pour le traitement des maladies respiratoires dans la dystrophie musculaire

(84) Designated Contracting States:
**AT BE BG CH CY CZ DE DK EE ES FI FR GB GR HR HU IE IS IT LI LT LU LV MC MT NL NO PL PT RO SE SI SK TR**

(43) Date of publication of application:
**14.10.2009 Bulletin 2009/42**

(73) Proprietor: **Santhera Pharmaceuticals (Schweiz) AG**
**4410 Liestal (CH)**

(72) Inventors:
- **Buyse, Gunnar**
**3020 Hevent (BE)**
- **Meier, Thomas**
**4056 Basel (CH)**

(74) Representative: **Grünecker, Kinkeldey, Stockmair & Schwanhäusser**
**Leopoldstrasse 4**
**80802 München (DE)**

(56) References cited:
**WO-A-2006/100017**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 108 366 B1

## Description

The present invention relates to 2,3-dimethoxy-5-methyl-6-(10-hydroxydecyl)-1,4-benzoquinone (idebenone) for treating and/or preventing respiratory illness associated with certain forms of muscular dystrophy.

## Background of the Invention

Duchenne muscular dystrophy (DMD) is a recessively inherited progressive form of muscle-wasting disease affecting ~1 in 3'000 boys. The reported incidence is 25/100,000 live male births worldwide. First signs of the disease become apparent when boys start to walk. Muscle wasting occurs initially in proximal and later in distal muscle groups leading to the loss of ambulation in teenage patients. Mutations in the dystrophin gene and absence of dystrophin protein ultimately lead to death of DMD patients at early adulthood, mainly because of respiratory or cardiac failures. Clinical measures to improve quality of life comprise orthopedic surgery and nighttime ventilation. Becker muscular dystrophy (BMD) is caused by different mutations of the same dystrophin gene but has a milder clinical course and the patients have a prolonged life expectancy when compared to DMD patients. Cellular processes underlying DMD-associated muscle wasting include the loss of skeletal muscle fibers and accompanying invasion by connective and adipose tissue. Progressive weakness of the skeletal musculature, cardiac involvement and respiratory insufficiency leads to early morbidity and mortality in DMD/BMD patients.

Both DMD and BMD are caused by mutations in the dystrophin gene. The dystrophin gene consists of 2700 kbp and is located on the X chromosome (Xp21.2, gene bank accession number: M18533). The 14 kbp long mRNA transcript is expressed predominantly in skeletal, cardiac and smooth muscle and to a limited extent in the brain. The mature dystrophin protein has a molecular weight of -427 kDa and belongs to the spectrin superfamily of proteins (Brown S.C., Lucy J.A. (eds), "Dystrophin", Cambridge University Press, 1997). While the underlying mutation in DMD leads to a lack of dystrophin protein, the milder BMD-phenotype is a consequence of mutations leading to the expression of abnormal, often truncated, forms of the protein with residual functionality.

The N-terminal part of dystrophin binds to actin filaments of the cytoskeleton, whereas domains in the C-terminal part of the dystrophin molecule bind to the membrane associated β-dystroglycan. Therefore, dystrophin serves as a molecular linker between the cytoskeleton and the muscle cell membrane and, indirectly, via the so-called dystrophin-associated protein complex (DAPC) also to the extracellular matrix. Known binding partners of dystrophin also include syntrophin, dystrobrevin, the neuronal type nitric oxide synthase (nNOS) and the sarcoglycan-sarcospan (SS) complex. These protein interactions involving both the carboxy- and aminoterminal region of the dystrophin protein are thought to contribute to the mechanical stability of the muscle cell membrane during cycles of contraction and relaxation. Dystrophin is also important for the assembly or integrity of the DAPC-complex itself, as it has been shown that in dystrophin-deficient muscle cells of DMD patients many components of the DAPC complex are reduced or absent in the sarcolemma. Absence of functional dystrophin protein leads to disruption of the mechanical link between actin cytoskeleton and the muscle cell sarcolemma which in turn leads to deterioration of myotubes and muscle weakness (Brown S.C., Lucy J.A. (eds), "Dystrophin", Cambridge University Press, 1997).

In dystrophin-deficient DMD patients respiratory illness results from weakness of respiratory muscles leading to a progressive restrictive pulmonary syndrome that becomes apparent in the first part of the second decade of life, and that results in respiratory insufficiency and life-threatening pulmonary infections during the second or third decade of life (McDonald CM, Abresch RT, Carter GT, Fowler WM, Johnson ER, Kilmer DD, Sigford BJ. Am J Phys Med Rehabil 1995;74 (Suppl): S70-92). In DMD patients progressive respiratory muscle weakness is the major factor in the development of respiratory insufficiency. Indeed, reduced maximal airway pressures have been shown to be the first sign of dysfunction, preceding the restrictive lung volume changes (Hahn A, Bach JR, Delaubier A, Renardel-Irani A, Guillou C, Rideau Y. Arch Phys Med Rehabil 1997;78:1-6; McDonald CM, Abresch RT, Carter GT, Fowler WM, Johnson ER, Kilmer DD, Sigford BJ. Am J Phys Med Rehabil 1995;74 (Suppl):S70-S92).

Respiratory insufficiency associated with DMD and BMD is currently treated only symptomatically or supportive by airway clearance, respiratory muscle training, non-invasive nocturnal ventilation, daytime non-invasive ventilation and continuous invasive ventilation as recommended by the American Thoracic Society. Spinal surgery in combination with nocturnal ventilation improves median survival to 30 years.

Pharmacological intervention for the treatment of DMD-associated muscle weakness is currently confined to the use of glucocorticoids such as prednisone or deflazacort. Conclusions of a recent review of available evidence on corticosteroids in DMD were as follows: prednisone (0.75 mg/kg/day) or deflazacort (0.9 mg/kg/day) should be offered as treatment, benefits and side effects should be monitored, and the offer of treatment with corticosteroids should include a balanced discussion of potential risks. Nevertheless, important questions or issues such as when to start corticosteroid treatment, and fear of significant side effects on the long-term remain.

WO 2006/100017 describes the use of idebenone in treating or preventing weakness and loss of skeletal muscle tissue or cardiomyopathy associated with a muscular dystrophy but is silent about the use for respiratory illness

in a muscular dystrophy.

In summary, despite the recent advances in managing respiratory insufficiency in DMD, respiratory complications and respiratory failure is a predominant cause of death in DMD. Accordingly, there is a strong need in the art to provide further means for treating or better preventing respiratory weakness and insufficiency in muscular dystrophies caused by dystrophin deficiency (Duchenne Muscular Dystrophy and Becker Muscular Dystrophy). Said object is achieved by providing idebenone for preparing a medicament for treating and/or preventing respiratory weakness and insufficiency associated with DMD and BMD.

**Description of the Invention**

The present invention relates to idebenone (2,3-dimethoxy-5-methyl-6-(10-hydroxydecyl)-1,4-benzoquinone) for the prophylaxis and/or treatment of respiratory illness (respiratory weakness andor insufficiency) in a muscular dystrophy, in particular Duchenne Muscular Dystrophy (DMD) and Becker Muscular Dystrophy (BMD). This is surprising since it has not been reported before that idebenone can ameliorate respiratory weakness or insufficiency in any neuromuscular disease.

Idebenone is a synthetic analogue of coenzyme Q10 (CoQ10), the vital cell membrane antioxidant and essential constituent of the adenosine-triphosphate (ATP)-producing mitochondrial electron transport chain (ETC). Idebenone has the ability to operate under low oxygen tension situations. Due to its ability to inhibit lipid peroxidation, idebenone protects cell membranes and mitochondria from oxidative damage (Zs.-Nagy I (1990) Chemistry, toxicology, pharmacology and pharmacokinetics of idebenone: a review. Arch. Gerontol. Geriatr. 11:177-186). It's antioxidant properties protect against cerebral ischemia and nerve damage in the central nervous system. Idebenone also interacts with the ETC, preserving ATP formation in ischemic states. This compound is already used as a nootropic drug and has also been shown to stimulate nerve growth factor, a characteristic that could be important in the treatment of Alzheimer's and other neurodegenerative diseases. Idebenone is described in the specification of Japanese Patent Examined Publication No. 3134/1987 filed by Takeda Chemical Industries, Ltd. In addition it has been shown that idebenone can be applied in the treatment of diseases associated with iron overload, particularly Friedreich Ataxia (US Patent 6,133,322).

Idebenone has the following formula:

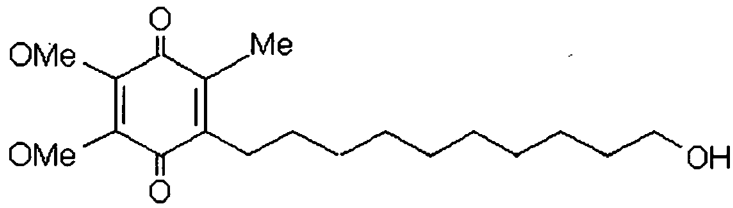

2,3-dimethoxy-5-methyl-6-(10-hydroxydecyl)-1,4-benzoquinone, idebenone

Idebenone is preferably administered in dosage ranges form 5 mg/kg/day to 60mg/kg/day, more preferably in a dosage range of 5 mg/kg/day to 40 mg/kg/day and most preferred in a dosage range of 10 mg/kg/day to 30 mg/kg/day.

Further, the idebenone is preferably administered at least one, preferably more times a day, preferably for at least 3 months, more preferably for at least 6 months, most preferably for 6 months to 12 months to observe the initial amelioration of muscle force and improved heart function and normalized heart anatomy. For maintenance of the therapeutic effect prolonged treatment is recommended; the preferred treatment is lifelong. Preferred modes of administration are oral, i.p., i.v., i.m., i.c, parenteral, intranasal, transdermal and oromucosal, whereas the oral and oromucosal administrations are the most preferred modes of administration.

Any suitable route of administration may be employed for providing a mammal, especially a human with an effective dosage of idebenone. Further modes of administration include rectal, topical, ocular, pulmonary or nasal administration. The dosage forms include, e.g., tablets, troches, dispersions, suspensions, solutions, capsules, creams, ointments and aerosols, whereas tablets are preferred.

Other preferred dosage forms are so called "wafers", defined as fast disintegrating oral film form products for the delivery of idebenone, or orally disintegrating tablets (ODTs) containing idebenone.

The effective dosage of the active ingredient employed may vary depending on the particular compounds employed, the mode of administration, the condition being treated and the severity of the condition being treated. Such dosage may be ascertained readily by a person skilled in the art, a preferred dosage having been mentioned above. Idebenone as used in the context of the present invention is preferably formulated into a dosage form prior to administration. Accordingly, the idebenone may be combined with any suitable pharmaceutical carrier. The pharmaceutical preparations for use in accordance with the present invention may be prepared by normal procedures using well-known

and readily available ingredients. In making the formulations, idebenone is usually mixed with a carrier, or diluted by a carrier, or enclosed with a carrier, which may be in the form of a capsule, cachet, paper or other container. When the carrier serves as a diluent, it may be a solid, semi-solid, or liquid material, which acts as a vehicle, excipient or medium for the active ingredient. The compositions can be in the form of tablets, pills, powders, lozenges, sachets, cachets, elixirs, suspensions, emulsions, solutions, syrups, aerosol (as a solid or in a liquid medium), soft and hard gelatin capsules, suppositories, sterile injectable solutions, sterile packaged powders, orally disintegrating tablets and oral wafers.

Some examples of suitable carriers, excipients and diluents include lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, water syrup, methyl cellulose, methyl and propylhydroxybenzoates, talc, magnesium stearate and mineral oil. The formulations can additionally include lubricating agents, wetting agents, emulsifying and suspending agents, preserving agents, sweetening agents and/or flavoring agents. The compositions of the invention may be formulated so as to provide quick, sustained or delayed release of the active ingredient after administration to the patient.

Idebenone is toxically safe which means that it can be used as a pharmaceutical active agent in a medicament.

Idebenone can be combined with excipients, fillers, solvents, diluents, dyes and/or binders. The choice of auxiliary substances as well as the amounts thereof to be used depends on whether the medicinal drug is to be administered orally, via the oral mucosa, intravenously, intraperitoneally, intradermally, intramuscularly, intranasally, buccally or topically. For oral application suitable preparations are in the form of tablets, sugar-coated pills, capsules, granular powders, drops, juices and syrups, while for parenteral, topical and inhalative application suitable forms are solutions, suspensions, easily reconstitutable dry preparations as well as sprays. The Idebenone can be administered in a sustained-release substance, in dissolved form or in a plaster, optionally with the addition of agents promoting penetration of the skin, and are suitable as percutaneous application preparations. Forms of preparations that can be used orally or percutaneously may produce a delayed release of the compounds. Idebenone formulations are e.g. described in several patents, for example in WO 99/07355, JP11116470 and WO 2008/019769.

Preferred formulations for use in accordance with the present invention contain 45 mg, 60 mg or 150 mg of Idebenone in a film-coated tablet containing lactose, cellulose, croscarmellose sodium, PVP (Plasdone® K25), magnesium stearate veg. and colloidal silicon dioxide.

Other preferred formulations are idebenone-containing wafers defined as fast disintegrating oral film form products for the delivery of idebenone or orally disintegrating tablet (ODT) containing idebenone.

In a further preferred embodiment, Idebenone may be administered in combination with a second therapeutic agent, wherein said second therapeutic agent is preferably selected from glucocorticosteroids such as 6a-methylprednisolone-21 sodium succinate (solumedrol®) or deflazacort (calcort®) which are routinely used in DMD patients for treatment of inflammation and muscle weakness. Likewise, idebenone may be administered in combination with any medication used in DMD patients to treat DMD-associated cardiomyopathy such as ACE-inhibitors, beta-blockers and diuretics.

Idebenone and the further active agent can be used simultaneously, separately or sequentially in order to treat or prevent the disease symptoms. The two active agents may be provided in a single dosage form or a separate formulation, each formulation containing at least one of the two active agents.

The following examples further illustrate the invention.

**Example 1**

Efficacy of idebenone on respiratory parameters in Duchenne Muscular Dystrophy (DMD) patients was assessed in a double-blind, placebo-controlled, randomised parallel group, clinical trial conducted in one singe clinical center. DMD patients at age 8 to 16 years were treated with idebenone or placebo over a period of 52 weeks. After written informed consent was obtained from the patient and the patient's parent/legal guardian, patients who met the protocol eligibility criteria were enrolled at the study centre and randomised to daily treatment of idebenone (150 mg, 3x daily; total daily dose of 450 mg) or placebo (3x daily). Efficacy was assessed at baseline and at weeks 26 and 52.

A total of 21 patients were enrolled, 13 patients were randomised to treatment with idebenone and 8 randomised to treatment with placebo.

The inclusion and exclusion criteria were assessed at Screening and confirmed at Visit 1 (baseline visit), prior to first administration of study medication. The following inclusion and exclusion criteria were used to decide on the eligibility for a patient to become enrolled into the study.

The inclusion criteria were:

- patients 8 - 16 years of age at time of enrolment
- male

- presence of cardiac involvement/dysfunction, defined by abnormal peak systolic strain in left ventricle (LV) inferolateral wall
- confirmed diagnosis of DMD (out of frame dystrophin gene deletion OR absent/<5% dystrophin protein on muscle biopsy; clinical picture consistent of typical DMD)
- if on chronic glucocorticosteroids treatment (deflazacort, prednisone) for DMD (or any other disease) (i.e. concomitant medication): dosage must be stable (unchanged) 6 months prior to inclusion
- if on chronic medication for DMD associated cardiomyopathy (β-blocker, diuretics): dosage must be stable (unchanged) 3 months prior to inclusion
- ability to provide reproducible repeat QMT upper limb score within 15% of first assessment score (at Visit1/Day 1 versus Screening Visit)

The exclusion criteria were:

- symptomatic cardiomyopathy or heart failure
- asymptomatic but severe cardiac dysfunction on baseline (Screening) evaluation: Fractional Shortening (FS) < 20% and/or Ejection Fraction (EF) < 40%
- Use of angiotensin converting enzyme (ACE) inhibitors
- previous history of ventricular arrhythmias (other than isolated ventricular extrasystole); ventricular arrhythmias presented at Screening
- previous (6 months or less) participation in any other therapeutic trial for DMD
- use of coenzymeQ10, idebenone, creatine, glutamine, oxatomide, or any herbal medicines within the last 6 months
- history of significant concomitant illness or significant impairment of renal or hepatic function (serum creatinine and GGT greater than 1.5 times upper limit for age and gender)
- known individual hypersensitivity to idebenone

The following patient elimination criteria were defined:

- use of any investigational drug other than the study medication during the study period

- administration of ACE inhibitors

- progressive cardiomyopathy during the trial, with patient developing symptomatic heart failure

- progressive cardiomyopathy during the trial, patient asymptomatic, but significant decrease of fractional shortening (FS) to < 20%, or ejection fraction (EF) to < 40%

Idebenone was formulated as a round biconvex shaped, film-coated orange tablet, with a 10 mm diameter, containing 150 mg of idebenone and Lactose Monohydrate, Microcrystalline Cellulose, Croscarmellose Sodium, Povidone (Plasdone® K25), Magnesium Stearate and Silicon Dioxide as excipients. The tablet is manufactured by a wet granulation process performed with a high-shear granulator, followed by compression on a rotary tabletting machine and film-coating of the cores in a perforated pan coater. The composition of the placebo is based on the composition of the 150 mg film-coated tablet. The drug substance and the excipients have been replaced by a mixture of lactose monohydrate and microcrystalline cellulose. The composition of the coating remained constant. Placebo tablets are manufactured by direct compression, followed by film-coating.
Both idebenone (150 mg) and matching placebo were administered as an oral tablet. Patients were to take 1 tablet, 3 times a day with meals, for 12 months (52 weeks).
Pulmonary function testing was performed by one clinical evaluator (physical therapist) with longstanding expertise in the assessment of children with neuromuscular diseases, using state of the art equipment and methods. Testing was done with the patient sitting, with a standardized order of testing. Lung volumes (FVC, FEV1) were measured using a handheld Koko spirometer (PDS Instrumentation, Louisville, USA).
Maximum inspiratory (MIP) and expiratory (MEP) pressures were measured using a Magnehelic® manometer (Dwyer Instrument, Michigan City Ind, USA) connected to a mouthpiece. Peak expiratory flow (PEF) measurements were performed using a portable peak flow meter (Mini-Wright peak flow meter, Clement Clarke International). For all parameters the highest values from three consecutive recordings were used in compliance with criteria published by the American Thoracic Society (ATS) (American Thoracic Society. Am J Respir Crit Care Med 1994;152:1107-1136).
Pulmonary function testing included the following parameters:

- Forced vital capacity (FVC) [L]

- Forced vital capacity % predicted
- Forced expiratory volume in 1 second (FEV1) [L]
- FEV1 (% predicted)
- Peak expiratory flow (PEF) [Umin]
- Peak expiratory flow (% predicted)
- Maximal Inspiratory Pressure (MIP) [$cmH_2O$]
- Maximal Inspiratory Pressure (% predicted)

For peak expiratory flow conversion from measured values (in Liter) to '% predicted' was performed by the following equation:

**"peak expiratory flow - 422,8 + 5,288 x height"**
**[height in cm]**

according to previous publications (Godfrey S et al. (1970) Lung volumes and airway resistance in normal children aged 5 to 18 years. Br J Dis Chest; 64(1):15-24; Quanjer PH, et al. (1989) Compilation of reference values for lung function measurements in children. Eur Respir J Suppl 4:184S-261S):

For maximal inspiratory pressure (MIP) conversion from measured values (in cm $H_2O$) to '% predicted' was performed by the following equation:

**"MIP- 27,020 - (4,132 x age) - (0,003 x height x weight)"**
**[age: years; height: cm body height; weight: kg]**

according to previous publications (Domènech-Clar R, et al. (2003) Maximal static respiratory pressures in children and adolescents. Pediatr Pulmonol. 35(2):126-32).

Efficacy of idebenone was determined for the parameters listed above as changes between baseline and end of treatment (at week 52) and comparisons were made between idebenone treated and placebo treated subjects.
The above described clinical testing allows the monitoring of any improvement on respiratory parameters in DMD patients as the result of the idebenone treatment.

**Example 2**

Idebenone improves functional respiratory parameters in patients with DMD.

To assess efficacy of idebenone to improve early signs of respiratory insufficiency in DMD, changes in peak expiratory flow (PEF) and maximal inspiratory pressure (MIP) between baseline and week 52 (end of treatment) was determined and the changes for idebenone and placebo groups compared. As shown in Table 1, patients on idebenone surprisingly improved for both parameters. Specifically, for patients on idebenone the peak expiratory flow was higher at week 52 compared to baseline (indicating improvement of respiratory function), while the peak expiratory flow of patients on placebo decreased over the study period (indicating a worsening of respiratory function). The difference for the change between baseline and week 52 between the idebenone and placebo groups was statistically significant.

**Table 1 Efficacy of idebenone compared to placebo on parameters of respiratory function in DMD patients**

| |
|---|
| Data represent values at baseline and end of treatment (week 52) as well as the comparison for the change between baseline and week 52 for idebenone and placebo treated patients |

(continued)

| Respiratory parameters | Baseline (BL) | | Week 52 | | Change Week 52 - BL | | p value |
|---|---|---|---|---|---|---|---|
| | Placebo N=8 | Idebenone N=13 | Placebo N=8 | Idebenone N=13 | Placebo N=8 | Idebenone N=13 | |
| **Respiratory function:** | | | | | | | |
| • FVC [L] | 1.93 ± 0.38 | 2.19 ± 0.58 | 2.08 ± 0.59 | 2.33 ± 0.67 | 0.15 ± 0.29 | 0.14 ± 0.31 | 0.471 |
| • FVC [% predicted] | 80.6 ± 28.87 | 76.5 ± 25.58 | 82.6 ± 37.11 | 75.3 ± 24.79 | 2.0 ± 11.0 | -1.2 ± 6.4 | 0.793 |
| • FEV1 [L] | 1.70 ± 0.39 | 1.97 ± 0.59 | 1.81 ± 0.52 | 2.08 ± 0.71 | 0.10 ± 0.27 | 0.11 ± 0.37 | 0.484 |
| • FEV1 [% predicted] | 81.9 ± 29.68 | 76.5 ± 23.94 | 81.5 ± 35.80 | 74.5 ± 23.88 | -0.4 ± 9.2 | -2.0 ± 9.2 | 0.651 |
| • Peak expiratory flow (L/min] | 243.8 ± 73.7 | 261.5 ± 93.9 | 230.0 ± 67.8 | 292.3 ± 90.6 | -13.8 ± 51.0 | 30.8 ± 54.4 | 0.039 |
| • Peak expiratory flow [% predicted] | 74.8 ± 26.49 | 74.1 ± 26.75 | 66.3 ± 25.38 | 76.9 ± 20.13 | -8.5 ± 1 3.8 | 2.8 ± 13.8 | 0.042 |
| • MIP [$cmH_2O$] | -39.4 ± 16.57 | -40.0 ± 16.58 | -41.9 ± 15.34 | -47.3 ± 14.09 | -2.5 ± 6.6 | -7.33 ± 13.0 | 0.173 |
| • MIP [% predicted] | 45.0 ± 18.69 | 39.2 ± 15.89 | 44.3 ± 16.66 | 44.1 ± 13.38 | -0.8 ± 7.3 | 4.8 ± 13.0 | 0.142 |

Data expressed as mean ± standard deviation
FVC (forced vital capacity); FEV1 (forced expiratory volume 1 second); MIP (maximal inspiratory pressure)
p-value: for comparison between idebenone and placebo groups (one-sided test)
n: number of subjects

The same surprising improvement on idebenone was detectable for the change between baseline and week 52 in "peak expiratory flow expressed as the percentage of the predicted value". The difference in the change of this parameter for the idebenone group was statistically different from the placebo group.

In addition, for patients on idebenone the maximum inspiratory pressure (MIP) increased (expressed as more negative number) over the 52 week treatment period indicating that idebenone treated DMD patients exert a larger inspiratory force. This change was clearly different from placebo (Table 1). When MIP was expressed as percent of the predicted value, patients on idebenone increased by 4.8±13.0 % while patients on placebo declined by -0.8± 7.3%, clearly indicating the therapeutic potential of idebenone on functional respiratory parameters.

Changes in FVC, FVC % predicted, FEV1 and FEV1 % predicted upon idebenone treatment were not different from placebo treatment. In fact, at baseline in both groups these parameters (restrictive lung volumes) were clearly less affected than peak flow and airway pressures. This is consistent with previous reports that showed that in the course of DMD disease changes in lung volumes do occur later than changes in more direct measures of reduced respiratory strength (Hahn A, Bach JR, Delaubier A, Renardel-Irani A, Guillou C, Rideau Y Arch Phys Med Rehabil 1997;78:1-6; McDonald CM, Abresch RT, Carter GT, Fowler WM, Johnson ER, Kilmer DD, Sigford BJ. Am J Phys Med Rehabil 1995; 74 (Suppl):S70-S92).

## Claims

1. Idebenone for use in the prophylaxis and/or treatment of respiratory weakness and/or insufficiency in a muscular dystrophy.

2. Idebenone for use according to claim 1, wherein the muscular dystrophy is Duchenne Muscular Dystrophy (DMD).

3. Idebenone for use according to claim 1, wherein the muscular dystrophy is Becker Muscular Dystrophy.

4. Idebenone for use according to any of claims 1 to 3, wherein the idebenone is administered in a dosage of from 5 mg/kg/day to 60 mg/kg/day.

5. Idebenone for use according to any of claims 1 to 4, wherein the idebenone is administered one or more times daily over at least 3 months.

6. Idebenone for use according to any of claims 1 to 5, wherein the mode of administration of idebenone is selected from oral, i.p., i.v., i.m., i.c., parenteral, intranasal, transdermal or via the oral mucosa.

7. Idebenone for use according to any of claims 1 to 5, wherein the idebenone is administered in a form of a tablet.

8. Idebenone for use according to any of claims 1 to 5, wherein the idebenone is administered by an oral wafer, a fast disintegrating oral film form product for the delivery of pharmaceutical ingredients or via orally disintegrating tablet.

9. Idebenone for use according to any of claims 1 to 8, wherein the idebenone is administered in combination with a second therapeutic agent, preferably selected from from glucocorticosteroids and from therapeutic agents for the treatment of DMD-associated cardiomyopathy.

10. Idebenone for use according to claim 9, wherein the glucocortocosteroids are selected from 6$\alpha$-methylprednisolone-21 sodium succinate and deflazacort.

11. Idebenone for use according to claim 9, wherein the therapeutic agents for the treatment of DMD-associated cardiomyopathy are selected from ACE-inhibitors, beta-blockers and diuretics.

**Patentansprüche**

1. Idebenon zur Verwendung in der Vorbeugung und/oder Behandlung von respirato-rischer Schwäche und/oder Insuffizienz bei Muskeldystrophie.

2. Idebenon zur Verwendung gemäß Anspruch 1, wobei es sich bei der Muskeldys-trophie um Muskeldystrophie vom Typ Duchenne (DMD) handelt.

3. Idebenon zur Verwendung gemäß Anspruch 1, wobei es sich bei der Muskeldys-trophie um Muskeldystrophie vom Typ Becker handelt.

4. Idebenon zur Verwendung gemäß einem der Ansprüche 1 bis 3, wobei das Idebe-non in einer Dosierung von 5 mg/kg/Tag bis 60 mg/kg/Tag verabreicht wird.

5. Idebenon zur Verwendung gemäß einem der Ansprüche 1 bis 4, wobei das Idebe-non einmal oder mehrfach täglich über mindestens 3 Monate verabreicht wird.

6. Idebenon zur Verwendung gemäß einem der Ansprüche 1 bis 5, wobei die Art der Verabreichung von Idebenon aus oral, i.p., i.v., i.m., i.c., parenteral, intranasal, transdermal oder über die Mundschleimhaut ausgewählt ist.

7. Idebenon zur Verwendung gemäß einem der Ansprüche 1 bis 5, wobei das Idebe-non in Form einer Tablette verabreicht wird.

8. Idebenon zur Verwendung gemäß einem der Ansprüche 1 bis 5, wobei das Idebe-non mittels eines oralen Wafers, eines sich schnell auflösenden Produkts in Form eines oralen Films zur Abgabe von pharmazeutischen Inhaltsstoffen oder über ei-ne sich oral auflösende Tablette verabreicht wird.

9. Idebenon zur Verwendung gemäß einem der Ansprüche 1 bis 8, wobei das Idebe-non in Kombination mit einem zweiten therapeutischen Wirkstoff, der vorzugswei-se aus Glukocorticosteroiden und aus therapeutischen Wirkstoffen für die Behand-lung von mit DMD assoziierte Kardiomyopathie ausgewählt ist, verabreicht wird.

10. Idebenon zur Verwendung gemäß Anspruch 9, wobei die Glukocorticosteroide aus 6$\alpha$-Methylprednisolon-21-natriumsuccinat und Deflazacort ausgewählt sind.

11. Idebenon zur Verwendung gemäß Anspruch 9, wobei die therapeutischen Wirk-stoffe für die Behandlung von mit DMD assoziierte Kardiomyopathie aus ACE-Hemmern, Beta-Blockern und Diuretika ausgewählt sind.

## Revendications

1. Idébénone utilisable dans la prophylaxie et/ou le traitement d'une faiblesse et/ou insuffisance respiratoire dans la myopathie primitive progressive.

2. Idébénone utilisable selon la revendication 1, la myopathie primitive progressive étant la myopathie de Duchenne.

3. Idébénone utilisable selon la revendication 1, la myopathie primitive progressive étant la myopathie pseudohypertrophique de Becker.

4. Idébénone utilisable selon l'une quelconque des revendications 1 à 3, l'idébénone étant administrée à une dose de 5 mg/kg/jour à 60 mg/kg/jour.

5. Idébénone utilisable selon l'une quelconque des revendications 1 à 4, l'idébénone étant administrée une ou plusieurs fois par jour sur une période d'au moins 3 mois.

6. Idébénone utilisable selon l'une quelconque des revendications 1 à 5, le mode d'administration de l'idébénone étant choisi parmi les voies orale, i.p., i. v., i.m., i.e., parentérale, intranasale, transdermique ou via la muqueuse orale.

7. Idébénone utilisable selon l'une quelconque des revendications 1 à 5, l'idébénone étant administrée sous la forme d'un comprimé.

8. Idébénone utilisable selon l'une quelconque des revendications 1 à 5, l'idébénone étant administrée par un cachet oral, un produit à usage oral sous forme de film à désagrégation rapide pour l'administration d'ingrédients pharmaceutiques ou via un comprimé à délitement oral.

9. Idébénone utilisable selon l'une quelconque des revendications 1 à 8, l'idébénone étant administrée en association avec un second agent thérapeutique, de préférence choisi parmi les glucocorticostéroïdes et parmi les agents thérapeutiques destinés au traitement des cardiomyopathies associées à la myopathie primitive progressive.

10. Idébénone utilisable selon la revendication 9, où les glucocorticostéroïdes sont choisis parmi la 6$\alpha$-méthylprednisolone-21-succinate de sodium et le déflazacort.

11. Idébénone utilisable selon la revendication 9, où les agents thérapeutiques destinés au traitement des cardiomyopathies associées à la myopathie primitive progressive sont choisis parmi les inhibiteurs d'ACE, les bêta-bloquants et les diurétiques.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2006100017 A
- JP 62003134 A
- US 6133322 A
- WO 9907355 A
- JP 11116470 B
- WO 2008019769 A


### Non-patent literature cited in the description


- Dystrophin. Cambridge University Press, 1997
- **McDonald CM ; Abresch RT ; Carter GT ; Fowler WM ; Johnson ER ; Kilmer DD ; Sigford BJ.** *Am J Phys Med Rehabil,* 1995, vol. 74, 70-92
- **Hahn A ; Bach JR ; Delaubier A ; Renardel-Irani A ; Guillou C ; Rideau Y.** *Arch Phys Med Rehabil,* 1997, vol. 78, 1-6
- **McDonald CM ; Abresch RT ; Carter GT ; Fowler WM ; Johnson ER ; Kilmer DD ; Sigford BJ.** *Am J Phys Med Rehabil,* 1995, vol. 74, S70-S92
- **Zs.-Nagy I.** Chemistry, toxicology, pharmacology and pharmacokinetics of idebenone: a review. *Arch. Gerontol. Geriatr.,* 1990, vol. 11, 177-186
- Am J Respir Crit Care Med. American Thoracic Society, 1994, vol. 152, 1107-1136
- **Godfrey S et al.** Lung volumes and airway resistance in normal children aged 5 to 18 years. *Br J Dis Chest,* 1970, vol. 64 (1), 15-24
- **Quanjer PH et al.** Compilation of reference values for lung function measurements in children. *Eur Respir J,* 1989, vol. 4, 184S-261S
- **Domènech-Clar R et al.** Maximal static respiratory pressures in children and adolescents. *Pediatr Pulmonol.,* 2003, vol. 35 (2), 126-32
- **Hahn A ; Bach JR ; Delaubier A ; Renardel-Irani A ; Guillou C ; Rideau Y.** *Arch Phys Med Rehabil,* 1997, vol. 78, 1-6